# NEUE EUROPÄISCHE PATENTSCHRIFT

(11) **EP 2 912 010 B2**
(45) Veröffentlichungstag und Bekanntmachung des Hinweises auf die Entscheidung über den Einspruch: **25.12.2019**
(45) Hinweis auf die Patenterteilung: 14.12.2016
(21) Anmeldenummer: 13780354.0
(22) Anmeldetag: 23.10.2013
(51) Int. Cl.: C07C 263/10

(54) **VERFAHREN ZUR HERSTELLUNG VON ISOCYANATEN DURCH PHOSGENIERUNG VON AMINEN IN FLÜSSIGER PHASE**
PROCESS FOR THE PREPARATION OF ISOCYANATES BY PHOSGENATION OF AMINES IN THE LIQUID PHASE
PROCÉDÉ DE FABRICATION D'ISOCYANATES PAR PHOSGÉNATION D'AMINES EN PHASE LIQUIDE

(30) Priorität: 24.10.2012 EP 12189765
(43) Veröffentlichungstag der Anmeldung: 02.09.2015
(73) Patentinhaber: BASF SE, 67056 Ludwigshafen am Rhein (DE)
(72) Erfinder: MATTKE, Torsten, 67251 Freinsheim (DE); HILLER, Markus, 67133 Maxdorf (DE); PALLASCH, Hans-Jürgen, 67169 Kallstadt (DE)
(86) Internationale Anmeldenummer: PCT/EP2013/072112
(87) Internationale Veröffentlichungsnummer: WO 2014/064128

(56) Entgegenhaltungen:
- EP-A1- 1 873 142
- US-A1- 2006 123 842
- US-A1- 2006 123 842
- US-A1- 2006 252 960
- US-A1- 2006 252 960
- US-A1- 2010 298 546
- ROBERT PERRY, CHEMICAL ENGINEERS' HANDBOOK, 1973, MacGraw-Hill, 6-29-6-32

## Beschreibung

Die Erfindung betrifft ein Verfahren zur Herstellung von aromatischen oder aliphatischen Isocyanaten durch Phosgenierung der entsprechenden Amine in flüssiger Phase.

Die Flüssigphasenphosgenierung von Aminen als klassische Route zur Herstellung von Isocyanaten ist vielfach beschrieben und wird im großtechnischen Maßstab durchgeführt (siehe z.B. Ullmanns Enzyklopädie der Technischen Chemie, Band 7 (Polyurethane), 3. neubearbeitete Auflage, Carl Hanser Verlag, München-Wien, S. 76 ff (1993).). Insbesondere die aromatischen Isocyanate TDI (Toluylendiisocyanat) und MDI (Methlyendiphenyldiisocyanat) sowie die aliphatischen Isocyanate Hexamethylendiisocyanat (HDI) und Isophorondiisocyanat (IPDI) werden großtechnisch global verteilt hergestellt.

Heutige großtechnische Synthesen der aromatischen Diisocyanate MDI und TDI sowie der aliphatischen Diisocyanate HDI und IPDI erfolgen fast ausschließlich in kontinuierlichen Verfahren.

Die Umsetzung der Edukte Amin und Phosgen erfolgt in der Regel mehrstufig. Ein derartiges mehrstufiges Verfahren mit sehr hoher chemischer Ausbeute, hoher Raum-Zeit-Ausbeute und niedrigem Phosgen-Holdup ist in der EP 1 575 904 beschrieben. Danach wird die Umsetzung zwischen einem organischen Amin und Phosgen drei- oder mehrstufig, in einem inerten Lösungsmittel, vorzugsweise Toluol, Chlorbenzol, Dichlorbenzol oder Mischungen der beiden letzteren, und mit einem Phosgenüberschuss durchgeführt, das dadurch gekennzeichnet ist, dass über jede der Stufen eine Reduzierung des Drucks erfolgt und die erste Phosgenierstufe einen statischen Mischer, bevorzugt eine Düse, umfasst, die zweite Stufe einen Verweilzeitapparat und die dritte Stufe in einer oder mehreren (Reaktions-)Kolonnen durchgeführt wird. Der Druck vor der Düse beträgt vorzugsweise 3 bis 70 bar. Der Verweilzeitreaktor der zweiten Stufe wird vorzugsweise bei einem Druck von 2,5 bis 35 bar betrieben. Hinter der Düse wird über eine Armatur oder eine andere dafür geeignete Einrichtung auf den Druck der zweiten Stufe entspannt. Es kann allerdings auch der natürliche Druckverlust der Düse oder der Verbindung von Düse und Verweilzeitreaktor zur Druckreduzierung verwendet werden. Der Reaktor der ersten Stufe, zumeist ein statischer Mischer, kann auch in den Reaktor der zweiten Stufe, einen Verweilzeitapparat, integriert werden. Der Reaktor der dritten Phosgenierstufe, eine Reaktionskolonne, wird vorzugsweise bei einem Druck von 2 bis 20 bar betrieben. Hinter dem Reaktor der zweiten Stufe wird über eine Armatur oder eine andere dafür geeignete Einrichtung auf den Druck des Reaktors der dritten Stufe entspannt. Gegebenenfalls ist auch ein natürlicher Druckverlust zur Druckreduzierung ausreichend.

Anschließend wird das verbleibende isocyanathaltige Produktgemisch einer weiteren Aufarbeitung (Lösungsmittelabtrennung, Reindestillation) zugeführt. Als Nebenprodukt anfallende HCl, überschüssiges Phosgen sowie Lösungsmittelreste werden aus den Reaktions- und Destillationsstufen als gasförmige Brüden abgezogen und einer thermischen Trennung, z.B. nach WO 2004056758, zugeführt.

Der Druck fällt begonnen mit Mischapparat, Verweilzeitreaktor, Reaktionskolonne, Phosgenstripper und HCl-Phosgen-Trennung stetig ab. Zur Vermeidung zu hoher und damit ausbeuteschädlicher Sumpftemperaturen im Phosgenstripper wird der Druck in dieser Kolonne möglichst klein gehalten (1 bis 4 bar). Über den Brüdenstrom ist die HCl-Phosgentrennung angekoppelt und muss entsprechend bei leicht geringerem Druck gefahren werden. Für die thermische Abtrennung von Phosgen aus dem HCl-Strom sind daher entsprechend tiefe Temperaturen erforderlich (-40 bis -20 °C). Die dafür erforderliche Kälteenergie führt jedoch zu hohen Investitions- und Betriebskosten.

Es war demgegenüber Aufgabe der Erfindung, ein verbessertes Verfahren zur Herstellung von Isocyanaten durch Flüssigphasenphosgenierung der entsprechenden Amine zur Verfügung zu stellen, das in technisch einfacher Weise zu deutlichen Einsparungen an Investitions- und Betriebskosten führt.

Die Aufgabe wird gelöst durch ein Verfahren zur Herstellung von Isocyanaten durch Umsetzung von Aminen mit Phosgen in flüssiger Phase, mit den folgenden Verfahrensstufen:
a) Mischung eines flüssigen aminhaltigen Eduktstromes und eines flüssigen phosgenhaltigen Eduktstromes unter Erhalt eines Zuführstromes,
b) Umsetzung des Zuführstromes aus der Verfahrensstufe a) in einem Verweilzeitreaktor, unter Erhalt eines zweiphasigen, gasförmigen/flüssigen Reaktionsgemisches, das unmittelbar oder nach Abtrennung der gasförmigen Phase in einem nachgeschalteten Abscheider einer Reaktionskolonne zugeführt wird,
c) Umsetzung des zweiphasigen, gasförmigen/flüssigen Reaktionsgemisches oder nur der flüssigen Phase hiervon in der Reaktionskolonne, unter Erhalt eines Kopfstromes und eines Sumpfstromes, der einer Phosgen/Chlorwasserstoff-Strippkolonne zugeführt wird,
d) destillative Abtrennung von Phosgen und Chlorwasserstoff über den Kopfstrom der Phosgen/Chlorwasserstoff-Strippkolonne, wobei in der Phosgen/Chlorwasserstoff-Strippkolonne ein Sumpfstrom erhalten wird, enthaltend das Isocyanat und das Lösungsmittel, woraus in Verfahrensstufe
e) durch destillative Abtrennung des Lösungsmittels das Wertprodukt Isocyanat gewonnen wird, und wobei in Verfahrensstufe
f) durch thermische Auftrennung der Brüden aus der Phosgen/Chlorwasserstoff-Strippkolonne in einer Trennkolonne über Kopf ein Chlorwasserstoff enthaltender Brüdenstrom und ein Sumpfstrom erhalten werden, der Phosgen und Lösungsmittel enthält, und der in die Verfahrensstufe a) recycliert wird,
das dadurch gekennzeichnet ist, dass die thermische Auftrennung in Verfahrensstufe f) bei einem höheren Kopfdruck der Trennkolonne gegenüber dem Kopfdruck in der Phosgen/Chlorwasserstoff-Strippkolonne durchgeführt wird der höhere Kopfdruck in der Trennkolonne (K3) gegenüber der Phosgen/Chlorwasserstoff-Strippkolonne (K2) realisiert wird, indem der Kopfstrom (6) aus der Phosgen/Chlorwasserstoff-Strippkolonne (K2), oder ein Brüdenstrom (10), der neben einem Kondensatstrom (11) durch Teilkondensation des Kopfstromes (6) aus der Phosgen/Chlorwasserstoff-Strippkolonne (K2) in einem zweiten Kondensator (W2) erhalten wird, vor der Zuführung desselben zur Trennkolonne (K3) in einem Ejektor (E) unter Erhalt eines Stromes (12) komprimiert wird, der in die Trennkolonne (K3) eingeleitet wird, wobei als Treibstrom für den Ejektor (E) der Kopfstrom (4) aus der Reaktionskolonne (K1) oder die Gasphase des teilkondensierten Kopfstromes (4) aus der Reaktionskolonne, bevorzugt die Gasphase des teilkondensierten Köpfstromes(4) aus der Reaktionskolonne (K1) genutzt wird.

Es hat sich überraschend gezeigt, dass die Trennkolonne, in der Chlorwasserstoff über Kopf und Phosgen über den Sumpfstrom abgetrennt wird, bei höherem Druck und entsprechend höheren Temperaturen betrieben werden kann, mit entsprechenden Energieeinsparungen in der Kälteeinlage.

Ein weiterer Vorteil einer Chlorwasserstoff-Phosgen-Trennung bei höherem Druck ergibt sich, wenn der Chlorwasserstoff für eine weitere Verarbeitung, beispielsweise in einem Oxichlorierungsverfahren zur Herstellung von 1,2-Dichlorethan, komprimiert werden muss. In diesem Fall verringert sich die notwendige Verdichtung und somit die entsprechenden Investitions- und Betriebskosten für den Kompressor.

Die Erfindung geht aus von einem Verfahren zur Herstellung von Isocyanaten wie in EP 157 5904 beschrieben.

Jedoch wird der Trennkolonne zur Auftrennung von Chlorwasserstoff und Phosgen bei erhöhtem Druck gegenüber der vorgeschalteten Phosgen/Chlorwasserstoff-Strippkolonne betrieben.

Die Phosgen/Chlorwasserstoff-Strippkolonne wird insbesondere bei einem Kopfdruck zwischen 1 und 4 bar Überdruck betrieben.

Zwischen der Phosgen/Chlorwasserstoff-Strippkolonne und der Trennkolonne ist bevorzugt keine Einrichtung zur aktiven Druckminderung vorgesehen.

Der höhere Kopfdruck in der Kolonne gegenüber der Phosgen/Chlorwasserstoff-Strippkolonne wird realisiert, indem der Kopfstrom aus der Phosgen/Chlorwasserstoff-Strippkolonne oder ein Brüdenstrom, der neben einem Kondensatstrom durch Teilkondensation des Kopfstromes aus der Phosgen/Chlorwasserstoff-Strippkolonne in einem Kondensator (W2) erhalten wird, vor der Zuführung desselben zur Trennkolonne in einem Ejektor (E) unter Erhalt eines Stromes komprimiert wird, der in die Trennkolonne (K3) eingeleitet wird.

Das Verfahren ist nicht beschränkt bezüglich der danach herstellbaren Isocyanate. Hierbei kann es sich bevorzugt um die aromatischen Isocyanate TDI (Toluylendiisocyanat) und MDI (Methlyendiphenyldiisocyanat) sowie die aliphatischen Isocyanate Hexamethylendiisocyanat (HDI) und Isophorondiisocyanat (IPDI) handeln.

Als Treibstrom für den Ejektor wird der Kopfstrom aus der Reaktionskolonne (K1) oder die Gasphase des teilkondensierten Kopfstromes aus der Reaktionskolonne, bevorzugt die Gasphase des teilkondensierten Kopfstromes aus der Reaktionskolonne (K1) genutzt.

Weiter bevorzugt wird der Kondensatstrom, der durch Teilkondensation des Kopfstromes aus der Phosgen/Chlorwasserstoff-Strippkolonne (K2) im Kondensator erhalten wird, in einem weiteren Ejektor unter Erhalt eines Kondensatstromes komprimiert, der in die Trennkolonne eingeleitet wird, wobei der Ejektor als Treibstrom den Kondensatstrom nutzt, der nach Teilkondensation des Kopfstromes aus der Reaktionskolonne im Kondensator neben dem Brüdenstrom erhalten wird. Der Kondensatstrom aus der Teilkondensation des Kopfstroms aus der Phosgen/Chlorwasserstoff-Strippkolonne kann jedoch mittels einer Pumpe in die auf höherem Kopfdruck arbeitende Trennkolonne gefördert werden.

Die Erfindung wird im Folgenden anhand einer Zeichnung sowie von Ausführungsbeispielen näher erläutert.

Die Zeichnungen zeigen im Einzelnen:
Figur 1 eine schematische Darstellung einer Anlage zur Herstellung von Isocyanaten durch Flüssigphasenphosgenierung von Aminen nach dem Stand der Technik,
Figur 2 eine schematische Darstellung einer bevorzugten Ausführungsform einer Anlage zur Durchführung des erfindungsgemäßen Verfahrens und

In den Figuren bezeichnen gleiche Bezugszeichen jeweils gleiche oder entsprechende Merkmale.

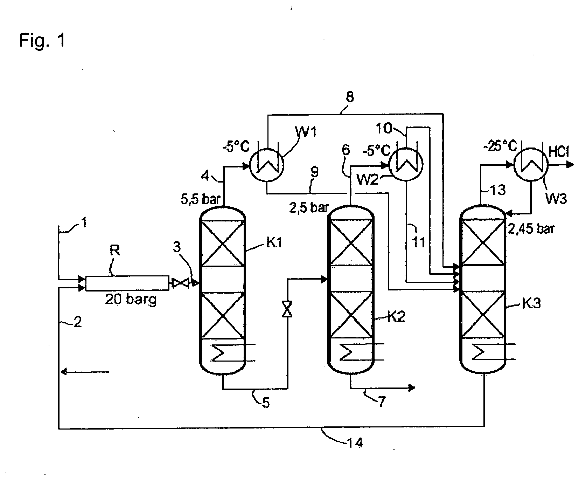

Die in Figur 1 dargestellte Anlage zur Durchführung einer Flüssigphasenphosgenierung nach dem Stand der Technik umfasst einen Verweilzeitreaktor R, dem ein aminhaltiger Strom 1 und ein phosgenhaltiger Strom 2 zugeführt wird. Das Reaktionsgemisch 3 aus dem Verweilzeitreaktor R wird einer Reaktionskolonne K1 zugeführt und darin in einen Kopfstrom 4 und einen Sumpfstrom 5 aufgetrennt. Der Kopfstrom 4 wird in einem ersten Kondensator W1 in einen Brüdenstrom 8 und einen Kondensatstrom 9 aufgetrennt, die jeweils einer Trennkolonne K3 zugeführt werden. Der Sumpfstrom 5 aus der Reaktionskolonne K1 wird einer Phosgen/Chlorwasserstoff-Strippkolonne K2 zugeführt, und darin in einen Strom 6 aufgetrennt, der in einem zweiten Kondensator W2 in einen Brüdenstrom 10 und einen Kondensatstrom 11 aufgetrennt wird, die jeweils der Trennkolonne K3 zugeführt werden, sowie in einen Sumpfstrom 7, enthaltend das Isocyanat und Lösungsmittel, woraus durch destillative Abtrennung des Lösungsmittels in einer in der Figur nicht dargestellten Einrichtung das Isocyanat gewonnen wird. In der Trennkolonne K3 wird ein Kopfstrom 13 abgezogen, der in einem dritten Kondensator W3 teilweise kondensiert wird, sowie ein Sumpfstrom 14, der in das Verfahren recycliert wird.

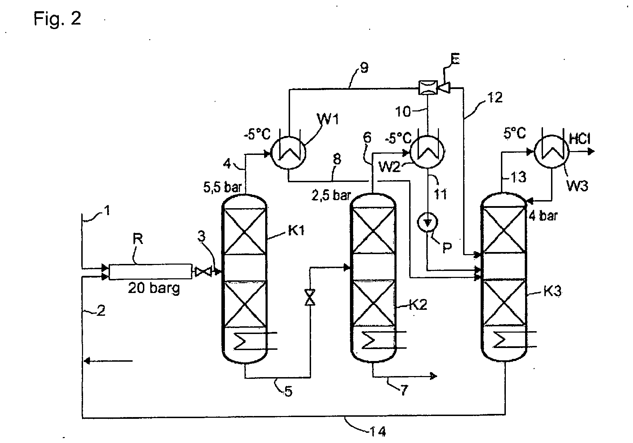

Das in Figur 2 dargestellte Schema einer bevorzugten erfindungsgemäßen Anlage umfasst gegenüber der in Figur 1 dargestellten Anlage nach dem Stand der Technik einen Ejektor E, worin der Brüdenstrom 10, der aus dem Kopfstrom 6 der Phosgen/- Chlorwasserstoff-Strippkolonne K2 im zweiten Kondensator W2 gewonnen wird, mittels des Brüdenstromes 9, der durch Teilkondensation des Kopfstromes 4 aus der Reaktionskolonne K1 im ersten Kondensator W1 gewonnen wird, komprimiert wird, unter Erhalt eines Stromes 12, der der Trennkolonne K3 zugeführt wird. In der In Figur 2 dargestellten bevorzugten Ausführungsvariante wird der Kondensatstrom 11, der durch Teilkondensation des Kopfstromes 6 aus der Phosgen/Chlorwasserstoff-Strippkolonne K2 im Kondensator zweiten W2 gewonnen wird, mittels einer Pumpe P der Trennkolonne K3 zugeführt.

### Vergleichsbeispiel

In einer entsprechend der in Figur 1 dargestellten Anlage zur Herstellung von Toluylendiisocyanat werden ein aminhaltiger Strom 1 und ein phosgenhaltiger Strom 2 in einem Verweilzeitreaktor R bei ca. 20 bar gemischt und das Amin reagiert im Verweilzeitreaktor R zum entsprechenden Isocyanat unter Bildung von HCl ab. In der anschließenden Reaktionskolonne K1 werden bei 5,5 bar gegebenenfalls gebildete Zwischenprodukte (Carbamoylchloride) thermisch in das Isocyanat und HCl gespalten. Über den Kopfstrom 4 der Reaktionskolonne K1 werden HCl und überschüssiges Phosgen abgezogen. Der Sumpfstrom 5 aus der Reaktionskolonne K1 wird einer Phosgen/Chlorwasserstoff-Strippkolonne K2 zugeführt, in der bei 2,5 bar Restspuren an HCl und Phosgen sowie Teile des Lösungsmittels abgezogen werden. Der Sumpfstrom 7 der Phosgen/Chlorwasserstoff-Strippkolonne K2 ist weitestgehend Phosgen- und HCl-frei. Die Kopfströme beider Kolonnen K1 und K2, Strom 4 und Strom 6, die im Wesentlichen HCl, Phosgen und Lösungsmittel enthalten, erden jeweils in nachgeschalteten Kondensatoren, W1 und W2, bei -5 °C teilkondensiert. Anschließend werden die Kondensatströme 9 und 11, und die Restbrüden, Ströme 8 und 10, einer weiteren Trennkolonne K3 zur Trennung von HCl und Phosgen bei 2,45 bar zugeführt. Hierzu muss am Kopf der Trennkolonne K3 im Kondensator W3 eine Kondensatortemperatur von -25 °C realisiert werden.

### Ausführungsbeispiel 1 (erfindungsgemäß)

In der in Figur 2 dargestellten Anlage werden in einer Treibstrahldüse E (Ejektor) die Restbrüden 10 aus der Phosgen/Chlorwasserstoff-Strippkolonne K2 mittels der Restbrüden 8 aus der Reaktionskolonne K1 auf einen Druck von 4 bar verdichtet und der Trennkolonne K3 zugeführt. Diese kann dadurch bei 4 bar anstatt bei 2,45 bar wie im Vergleichsbeispiel betrieben werden. Das Kondensat aus der Phosgen/Chlorwasserstoff-Strippkolonne K2 wird mittels einer Pumpe P ebenfalls auf 4 bar gebracht. Durch den erhöhten Kolonnendruck steigt die Kondensationstemperatur für den Brüdenstrom 13 der Trennkolonne K3 auf -5 °C. Die im Vergleichsbeispiel erforderliche Tieftemperaturkondensation bei -25 °C entfällt in diesem Fall, und wird durch Kälte auf höherem und damit preiswerterem Niveau ersetzt.

## Patentansprüche

1. Verfahren zur Herstellung von Isocyanaten durch Umsetzung von Aminen mit Phosgen in flüssiger Phase, mit den folgenden Verfahrensstufen:
a) Mischung eines flüssigen aminhaltigen Eduktstromes (1) und eines flüssigen phosgenhaltigen Eduktstromes (2) unter Erhalt eines Zuführstromes,
b) Umsetzung des Zuführstromes aus der Verfahrensstufe a) in einem Verweilzeitreaktor (R), unter Erhalt eines zweiphasigen, gasförmigen/flüssigen Reaktionsgemisches (3), das unmittelbar oder nach Abtrennung der gasförmigen Phase in einem nachgeschalteten Abscheider (A) einer Reaktionskolonne (K1) zugeführt wird,
c) Umsetzung des zweiphasigen, gasförmigen/flüssigen Reaktionsgemisches (3) oder nur der flüssigen Phase hiervon in der Reaktionskolonne (K1), unter Erhalt eines Kopfstromes (4) und eines Sumpfstromes (5),der einer Phosgen/Chlorwasserstoff-Strippkolonne (K2) zugeführt wird,
d) destillative Abtrennung von Phosgen und Chlorwasserstoff über den Kopfstrom (6) der Phosgen/Chlorwasserstoff-Strippkolonne (K2), wobei in der Phosgen/- Chlorwasserstoff-Strippkolonne (K2) ein Sumpfstrom (7) erhalten wird, enthaltend das Isocyanat und das Lösungsmittel, woraus in Verfahrensstufe
e) durch destillative Abtrennung des Lösungsmittels das Wertprodukt Isocyanat gewonnen wird, und wobei in Verfahrensstufe
f) durch thermische Auftrennung der Brüden aus der Phosgen/Chlorwasserstoff-Strippkolonne (K2) in einer Trennkolonne (K3) über Kopf ein Chlorwasserstoff enthaltender Brüdenstrom (13) und ein Sumpfstrom (14) erhalten werden, der Phosgen und Lösungsmittel enthält, und der in die Verfahrensstufe (a) recycliert wird,
**dadurch gekennzeichnet, dass** die thermische Auftrennung in Verfahrensstufe f) bei einem höheren Kopfdruck der Trennkolonne (K3) gegenüber dem Kopfdruck in der Phosgen/Chlorwasserstoff-Strippkolonne (K2) durchgeführt wird und der höhere Kopfdruck in der Trennkolonne (K3) gegenüber der Phosgen/Chlorwasserstoff-Strippkolonne (K2) realisiert wird, indem der Kopfstrom (6) aus der Phosgen/Chlorwasserstoff-Strippkolonne (K2), oder ein Brüdenstrom (10), der neben einem Kondensatstrom (11) durch Teilkondensation des Kopfstromes (6) aus der Phosgen/Chlorwasserstoff-Strippkolonne (K2) in einem zweiten Kondensator (W2) erhalten wird, vor der Zuführung desselben zur Trennkolonne (K3) in einem Ejektor (E) unter Erhalt eines Stromes (12) komprimiert wird, der in die Trennkolonne (K3) eingeleitet wird, wobei als Treibstrom für den Ejektor (E) der Kopfstrom (4) aus der Reaktionskolonne (K1) oder die Gasphase des teilkondensierten Kopfstromes (4) aus der Reaktionskolonne, bevorzugt die Gasphase des teilkondensierten Kopfstromes(4) aus der Reaktionskolonne (K1) genutzt wird.

2. Verfahren nach Anspruch 1, **dadurch gekennzeichnet, dass** der Kondensatstrom (11), der durch Teilkondensation des Kopfstromes (6) aus der Phosgen/- Chlorwasserstoff-Strippkolonne (K2) im zweiten Kondensator (W2) erhalten wird, in einem weiteren Ejektor (E) unter Erhalt eines Kondensatstromes komprimiert wird, der in die Trennkolonne (K3) eingeleitet wird, wobei der weitere Ejektor (E) als Treibstrom den Kondensatstrom (8) nutzt, der nach Teilkondensation des Kopfstromes (4) aus der Reaktionskolonne (K1) im ersten Kondensator (W1) neben dem Brüdenstrom (9) erhalten wird.

3. Verfahren nach einem der Ansprüche 1 bis 2, **dadurch gekennzeichnet, dass** der Kopfdruck in der Phosgen/Chlorwasserstoff-Strippkolonne (K2) zwischen 1 und 4 bar Überdruck beträgt.

## Claims

1. A process for preparing isocyanates by reaction of amines with phosgene in the liquid phase, which comprises the following process stages:
a) mixing of a liquid amine-comprising starting material stream (1) and a liquid phosgene-comprising starting material stream (2) to give a feed stream,
b) reaction of the feed stream from process stage a) in a residence reactor (R) to give a two-phase, gaseous/liquid reaction mixture (3) which is fed either directly or after removal of the gaseous phase in a downstream separator (A) to a reaction column (K1),
c) reaction of the two-phase, gaseous/liquid reaction mixture (3) or only the liquid phase thereof in the reaction column (K1) to give an overhead stream (4) and a bottom stream (5) which is fed to a phosgene/hydrogen chloride stripping column (K2),
d) removal by distillation of phosgene and hydrogen chloride via the overhead stream (6) from the phosgene/hydrogen chloride stripping column (K2), giving a bottom stream (7) from the phosgene/hydrogen chloride stripping column (K2) which comprises the isocyanate and the solvent, from which, in process stage
e) the desired isocyanate product is obtained by removal of the solvent by distillation and, in process stage
f) thermal separation of the vapor from the phosgene/hydrogen chloride stripping column (K2) in a separation column (K3) gives a hydrogen chloride-comprising vapor stream (13) at the top and a bottom stream (14) which comprises phosgene and solvent and is recycled to process stage (a),
wherein the thermal separation in process stage f) is carried out at a pressure at the top of the separation column (K3) which is higher than the pressure at the top of the phosgene/hydrogen chloride stripping column (K2) and the higher pressure at the top of the separation column (3) compared to the phosgene/hydrogen chloride stripping column (K2) is achieved by the overhead stream (6) from the phosgene/hydrogen chloride stripping column (K2) or a vapor stream (10) obtained together with a condensate stream (11) by partial condensation of the overhead stream (6) from the phosgene/hydrogen chloride stripping column (K2) in a second condenser (W2) being compressed in an ejector (E) before being fed to the separation column (K3) so as to give a stream (12) which is introduced into the separation column (K3), where the overhead stream (4) from the reaction column or the gas phase of the partially condensed overhead stream (4) from the reaction column, preferably the gas phase of the partially condensed overhead stream (4) from the reaction column (K1), is utilized as driving stream for the ejector (E).

2. The process according to claim 1, wherein the condensate stream (11) which is obtained by partial condensation of the overhead stream (6) from the phosgene/hydrogen chloride stripping column (K2) in the second condenser (W2) is compressed in a further ejector (E) to give a condensate stream which is introduced into the separation column (K3), with the further ejector (E) utilizing, as driving stream, the condensate stream (8) which is obtained in addition to the vapor stream (9) after partial condensation of the overhead stream (4) from the reaction column (K1) in the first condenser (W1).

3. The process according to either of claims 1 and 2, wherein the pressure at the top of the phosgene/hydrogen chloride stripping column (K2) is in the range from 1 to 4 bar gauge.

## Revendications

1. Procédé pour la préparation d'isocyanates par la transformation d'amines avec du phosgène en phase liquide, comportant les étapes de procédé suivantes :
a) mélange d'un flux d'éduit liquide (1) contenant une amine et d'un flux d'éduit liquide (2) contenant du phosgène avec obtention d'un flux d'introduction,
b) transformation du flux d'introduction de l'étape de procédé a) dans un réacteur (R) de temps de séjour, avec obtention d'un mélange réactionnel (3) biphasique, gazeux/liquide, qui est introduit, immédiatement ou après la séparation de la phase gazeuse, dans un séparateur (A), monté en aval, d'une colonne de réaction (K1),
c) transformation du mélange réactionnel (3) biphasique, gazeux/liquide ou seulement de la phase liquide de celui-ci dans la colonne de réaction (K1), avec obtention d'un flux de tête (4) et d'un flux de fond (5), qui est introduit dans une colonne de rectification (K2) de phosgène/chlorure d'hydrogène,
d) séparation par distillation de phosgène et de chlorure d'hydrogène sur le flux de tête (6) de la colonne de rectification (K2) de phosgène/chlorure d'hydrogène, dans la colonne de rectification (K2) de phosgène/chlorure d'hydrogène un flux de fond (7) étant obtenu, lequel contient l'isocyanate et le solvant, dans laquelle dans l'étape de procédé
e) le produit recherché de type isocyanate est obtenu par séparation par distillation du solvant, et dans l'étape de procédé
f) un flux de vapeurs (13) contenant du chlorure d'hydrogène et un flux de fond (14), qui contient du phosgène et du solvant, et qui est recyclé dans l'étape de procédé (a), étant obtenus par séparation thermique des vapeurs de la colonne de rectification (K2) de phosgène/chlorure d'hydrogène dans une colonne de séparation (K3) sur la tête,
**caractérisé en ce que** la séparation thermique dans l'étape de procédé f) est réalisée à une pression de tête de la colonne de séparation (K3) plus élevée que la pression de tête dans la colonne de rectification (K2) de phosgène/chlorure d'hydrogène et la pression de tête plus élevée dans la colonne de séparation (K3) que la pression de tête dans la colonne de rectification (K2) de phosgène/chlorure d'hydrogène est réalisée par l'opération dans laquelle le flux de tête (6) de la colonne de rectification (K2) de phosgène/chlorure d'hydrogène, ou un flux de vapeurs (10), qui est obtenu, à côté d'un flux de condensat (11), par condensation partielle du flux de tête (6) de la colonne de rectification (K2) de phosgène/chlorure d'hydrogène dans un deuxième condensateur (W2), avant l'acheminement de celui-ci même à la colonne de séparation (K3), est comprimé dans un éjecteur (E) avec obtention d'un flux (12), qui est engagé dans la colonne de séparation (K3), le flux de tête (4) de la colonne de réaction ou la phase gazeuse du flux de tête (4) partiellement condensé de la colonne de réaction, préférablement la phase gazeuse du flux de tête (4) partiellement condensé de la colonne de réaction (K1), est utilisé en tant que flux d'attaque pour l'éjecteur (E).

2. Procédé selon la revendication 1, **caractérisé en ce que** le flux de condensat (11), qui est obtenu par condensation partielle du flux de tête (6) de la colonne de rectification (K2) de phosgène/chlorure d'hydrogène dans le deuxième condensateur (W2), est comprimé dans un autre éjecteur (E) avec obtention d'un flux de condensat, qui est introduit dans la colonne de séparation (K3), l'autre éjecteur (E) utilisant le flux de condensat (8) en tant que flux d'attaque, lequel flux de condensat est obtenu après condensation partielle du flux de tête (4) de la colonne de réaction (K1) dans le premier condensateur (W1) à côté du flux de vapeurs (9).

3. Procédé selon l'une quelconque des revendications 1 et 2, **caractérisé en ce que** la pression de tête dans la colonne de rectification (K2) de phosgène/chlorure d'hydrogène est comprise entre 1 et 4 bars de surpression.
